# EUROPÄISCHE PATENTSCHRIFT

(11) **EP 0 661 060 B1**
(45) Veröffentlichungstag und Bekanntmachung des Hinweises auf die Patenterteilung: **23.05.2001**
(21) Anmeldenummer: 94120595.7
(22) Anmeldetag: 23.12.1994
(51) Int. Cl.: A61K 39/395

(54) **Hochkonzentriertes Immunglobulin-Präparat und Verfahren zu seiner Herstellung**
Highly concentrated immunoglobulin composition and method to prepare it
Composition d'immunoglobuline de haute concentration et procédé de sa préparation

(30) Priorität: 28.12.1993 DE 4344824
(43) Veröffentlichungstag der Anmeldung: 05.07.1995
(73) Patentinhaber: Baxter Aktiengesellschaft, 1221 Wien (AT)
(72) Erfinder: Eibl, Johann, Dr., A-1180 Wien (AT); Linnau, Yendra, Dr., A-1224 Wien (AT); Teschner, Wolfgang, Dr., A-1030 Wien (AT)
(74) Vertreter: Kolb, Helga, Dr. Dipl.-Chem.

(56) Entgegenhaltungen:
- EP-A- 0 168 506
- EP-A- 0 196 761
- EP-A- 0 352 500
- CH-A- 684 164
- GB-A- 2 030 150
- US-A- 4 186 192

## Beschreibung

Die Erfindung betrifft ein stabiles hochkonzentriertes, intravenös verträgliches Immunglobulin-Präparat.

Immunglobuline werden zur Behandlung einer Reihe von infektiösen und immunregulatorischen Krankheitszuständen an Patienten verabreicht. Durch die intravenöse Verabreichung ist die Versorgung der Patienten mit einer ausreichenden Immunglobulinmenge möglich. Dabei ist die Verwendung von Präparationen mit einer maximalen Konzentration von 5 % oder 6 % üblich . Im Hinblick auf die höhere Sicherheit der Infusion sind aber höherkonzentrierte Präparate wünschenswert. Durch die Verwendung eines geringeren Volumens könnte die Infusionszeit wesentlich verkürzt werden.

R.I. Schiff et al., J. Allergy Clin.Immunol. 88 (1991) 61-67 beschreiben ein lyophilisiertes Präparat, das zu einer 3, 6, 9 und 12%-igen Lösung rekonstituiert wurde. Es wurde gefunden, daß die Osmolarität der Lösungen sehr hoch liegt. Z.B. wies eine 12%-ige Lösung eine Osmolarität von 1074 mOs/l auf. Gleichzeitig wurde beobachtet, daß die Viskosität der Präparation ebenfalls sehr hoch war. Eine 12%-ige Lösung konnte daher nur schwer genügend rasch an Kinder verabreicht werden. Es wird angegeben, daß Konzentrationen von mehr als 12 % für eine routinemäßige Anwendung zu viskos sind.

Die Osmolarität einer isotonen Lösung, d.h. also eine Lösung, die den gleichen osmotischen Druck wie das menschliche Blut besitzt, beträgt ca. 300 mOs/l. Um eine gute Verträglichkeit eines intravenös verabreichbaren Präparates zu gewährleisten ist es daher wünschenswert daß dieses eine Osmolarität von unter 1000 mOs/l, und idealerweise einen der Isotonie entsprechenden Wert, aufweist.

Weiters muß bei intravenös verträglichen Immunglobulinpräparaten das Auftreten von IgG-Aggregaten verhindert werden. Diese führen nämlich zu einer unerwünschten antikomplementären Aktivität und damit verbunden zu einer Reihe von Nebenwirkungen. Daher wird bei der Formulierung eines Präparates darauf geachtet, daß dieses durch ein Verfahren hergestellt ist, das die Entfernung derartiger Aggregate bzw. die Verhinderung der Aggregatbildung beinhaltet. Nach Formulierung des Präparats wird es bevorzugt durch Lyophilisieren haltbar gemacht. A.M. Herrera et al., J. Allergy Clin.Immunol. 84 (1989) 556-561 vermuten jedoch, daß es durch die Lyophilisierung von Immunglobulinpräparaten wieder zur Aggregatbildung kommt.

Aufgabe der vorliegenden Erfindung ist die Bereitstellung eines stabilen Immunglobulin-Präparates, mit dem die vorstehend genannten Nachteile weitgehend überwunden werden können, und das in hochkonzentrierter Form verabreicht werden kann.

Diese Aufgabenstellung wird mit dem Gegenstand der vorliegenden Erfindung gelöst.

Gegenstand der Erfindung ist ein stabiles, hochkonzentriertes, intravenös verträgliches Immunglobulin-Präparat gemäß Anspruch 1.

Zweckmäßige Ausgestaltungen davon sind Gegenstand der Ansprüche 2 bis 10.

Weiterer Gegenstand der Erfindung ist ein Verfahren gemäß Anspruch 11 zur Herstellung eines stabilen, hochkonzentrierten, intravenös verträglichen Immunglobulin-Präparates.

Zweckmäßige Ausgestaltungen dieses Verfahrens sind Gegenstand der Ansprüche 12 und 13.

Das erfindungsgemäße Immunglobulin-Präparat weist einen Immunglobulin-Gehalt von 13.5 bis 17.5 % (G/V), und vorzugsweise von 14 bis 16 % (G/V) auf, und besitzt eine Osmolarität im Bereich von 250 bis 600 mOs/l und eine Viskosität von nicht mehr als 9 cP, vorzugsweise von 3-8 cP, auf.

Überraschenderweise hat es sich gezeigt, daß trotz Herabsetzen der Osmolarität die Herstellung eines stabilen, hochkonzentrierten Immunglobulin-Präparat möglich ist, ohne den Nachteil einer hohen Viskosität in Kauf nehmen zu müssen. Das erfindungsgemäße Präparat kann aufgrund seiner geringen Viskosität rasch an Patienten verabreicht werden, was vor allem bei Kindern mit schmalen Venen von großer Bedeutung ist.

Vorzugsweise besitzt das erfindungsgemäße Immunglobulin-Präparat einen pH-Wert im Bereich von 4 bis 8. Zur Einstellung des pH-Werts ist es zweckmäßig, daß die erfindungsgemäße Präparatformulierung einen Puffer mit einem pH-Wert im neutralen Bereich, oder auch im schwach sauren Bereich enthält.

Die Immunglobuline sind im erfindungsgemäßen Präparat vorteilhafterweise in nativer Form enthalten, d.h. in Form von Immunglobulinen, die weder fragmentiert noch chemisch modifiziert sind. Vorzugsweise werden Immunglobuline eingesetzt, die im wesentlichen aus IgG bestehen oder einen hohen Gehalt an IgG aufweisen.

Die Osmolarität des Präparates kann vor allem durch einen Gehalt an Kohlenhydraten, insbesondere Sacchariden, wie Glucose und Saccharose, an Glycin und/oder Natriumchlorid beeinflußt und eingestellt werden. Die Kohlenhydrate besitzen darüberhinaus auch eine Stabilisatorfunktion.

Zweckmäßigerweise enthält das erfindungsgemäße Immunglobulin-Präparat deshalb zur Stabilisierung und/oder Einstellung seiner physikalischen Parameter, wie insbesondere der Osmolarität der Präparateformulierung, einen Gehalt an Sacchariden. Der Gehalt an Sacchariden wird dabei vorzugsweise so gewählt, daß die Osmolarität im erfindungsgemäßen Bereich von 250 bis 600 mOs/l möglichst niedrig gehalten wird, und idealerweise die Osmolarität einer isotonen Lösung, also ca. 300 mOs/l, aufweist. Der Gehalt an Sacchariden, wie z.B. an Glucose oder Saccharose, liegt dabei vorzugsweise im Bereich von 30 bis 50 g/l. Es war nicht zu erwarten, daß in einem hochkonzentrierten Immunglobulin-Präparat mit einem Immunglobulin-Gehalt von 13.5 bis 17.5 % (G/V) ein derartig geringer Gehalt an Stabilisatoren, wie z.B. an Sacchariden entsprechend der geringen Osmolarität, ausreicht, um das Immunglobulin-Präparat mit einem Gehalt an nativem Immunglobulin und in flüssigem Zustand ausreichend zu stabilisieren. Durch diesen geringen Gehalt an Stabilisatoren ist es aber möglich, Immunglobulin-Präparate mit der gewünschten niedrigen Viskosität bereitzustellen.

Zur Einstellung des bevorzugten Gehalts an Stabilisatoren, insbesondere an Sacchariden, werden dem Immunglobulin-Präparat entweder entsprechende Mengen dieser Substanzen zugesetzt, oder aber dem als Ausgangsmaterial eingesetzten Präparat entzogen, wenn dieses bereits einen höheren Gehalt an Stabilisatoren, wie z.B. Sacchariden, enthält.

Das erfindungsgemäße Immunglobulin-Präparat kann als Flüssigpräparat, in tiefgefrorener Form oder in lyophilisierter Form vorliegen. Wenn von einer immunglobulinhaltigen Fraktion als Ausgangsmaterial ausgegangen wird, die frei von IgG-Aggregaten ist,
kann das Präparat zweckmäßigerweise zur weiteren Haltbarmachung einer Lyophilisation unterworfen werden, ohne die antikomplementäre Aktivität im Präparat wesentlich zu erhöhen.

In einer bevorzugten Ausführungsform liegt das erfindungsgemäße Immunglobulin-Präpart in einer durch eine Behandlung zur Virusinaktivierung erhältlichen, bezüglich von durch Blut übertragbare infektiöse Agentien sicheren Form vor.

Die Behandlung zur Virusinaktivierung erfolgt dabei zweckmäßigerweise bevor man das als Ausgangsmaterial verwendete Immunglobulinpräparat auf den erfindungsgemäßen Immunglobulin-Gehalt und Saccharid-Gehalt einstellt.

Zur Virusinaktivierung wird vorzugsweise eine chemische Behandlung vorgenommen, z.B. mit Ethanol bei Temperaturen von weniger als 0°C im sauren Milieu. Die chemische Behandlung wird dabei vorzugsweise in Gegenwart von Stabilisatoren, wie z.B. einem Polyethylenglycol, durchgeführt.

Das Verfahren zur Herstellung des erfindungsgemäßen Immunglobulin-Präparates kann auf an sich bekannte Weise durchgeführt werden, z.B. indem man das Immunglobulin-haltige Ausgangsmaterial, das vorzugsweise in lyophilisierter Form vorliegt, zur Einstellung des entsprechenden Immunglobulin-Gehaltes in Wasser löst, und den Gehalt an Stabilisatoren, wie z.B. an Glycin und/oder Natriumchlorid, und insbesondere an Sacchariden, durch Zugabe entsprechender Mengen oder durch eine Reduzierung, wie z.B. durch eine Diafiltration oder Dialyse, auf den zum Erhalt der gewünschten Osmolarität und Viskosität auf den erfindungsgemäßen Wert einstellt. Vor oder nach dieser Einstellung werden zweckmäßigerweise gegebenenfalls vorhandene IgG-Aggregate auf an sich bekannte Weise entfernt. Die erhaltene Endformulierung wird schließlich einer Sterilfiltration unterworfen.

Mit den erfindungsgemäßen Immunglobulin-Präparaten werden Präparate zur Verfügung gestellt, die eine hervorragende Haltbarkeit von mindestens 2 Jahren vorteilhafterweise von mindestens 4 Jahren, aufweisen.

Die erfindungsgemäßen, in Form von Flüssigpräparaten vorliegenden Präparate sind außerdem äußerst anwendungsfreundlich, weil sie vor ihrer Anwendung nicht rekonstituiert werden müssen.

Die Erfindung wird nun durch die nachfolgenden Beispiel näher erläutert, ohne sie darauf zu beschränken.

Wenn nicht anders angegeben, beziehen sich Prozentangaben auf das Verhältnis Gewicht/Volumen (G/V).

### Beispiele

### Beispiel 1

Ein Immunglobulin-haltiges lyophilisiertes Präparat (Endobulin^{R}, IMMUNO AG) wurde in einem Drittel des empfohlenen Volumens mit destilliertem Wasser gelöst. Der Gehalt an Glucose und Natriumchlorid wurde durch Diafiltration entsprechend eingestellt und die Lösung sterilfiltriert. Die infusionsbereite Lösung hatte folgende Zusammensetzung: 150 g/l Immunglobulin, 40 g/l Glucose, 3 g/l Natriumchlorid. Die Viskosität der Lösung wurde an einem BROOKFIELD-Viskosimeter und die Osmolarität an einem KNAUER-Osmometer gemessen. Das Präparat besaß die folgende Eigenschaften:

| | |
|---|---|
| pH-Wert | 7,0 |
| Viskosität | 7.6 cP |
| Osmolarität | 365 mOs/l |
| | |
| Antikomplementäre Aktivität | Sofortwert 19.6 CH₅₀ (bestimmt nach der Europ. Pharmacopoeia), nach 30 Monaten Lagerung bei 4 °C: 20,5 CH₅₀ |
| Monomere und Dimere | Sofortwert über 96 % (gemessen mittels HPLC), nach 30 Monaten Lagerung bei 4 °C: über 95 % |
| reines Gammaglobulin | 97 % (elektrophoretisch bestimmt) |

### Beispiel 2

6 x 1000 mg Sandoglobulin (Fa. SANDOZ) wurden in 40 ml dest. Wasser gelöst und gegen 4 l einer 0.3%igen NaCl-Lösung dialysiert. Zum Dialysat wurden 40 mg/ml Saccharose zugesetzt und anschließend einer Sterilfiltration unterworfen. Die Lösung ist zur i.v.-Anwendung geeignet; sie besitzt folgende Eigenschaften:

| | |
|---|---|
| pH-Wert | 6.9 |
| Viskosität | 7.5 cP |
| Osmolarität | 388 mOs/l |
| Antikomplementäre Aktivität | 23.1 CH₅₀ |

## Patentansprüche

1. Stabiles, hochkonzentriertes, intravenös verträgliches Immunglobulin-Präparat, dadurch gekennzeichnet, dass das Präparat einen Immunglobulin-Gehalt von 13,5 bis 17,5 % (G/V) aufweist, der Gehalt an Sacchariden so gewählt ist, dass die Osmolarität im Bereich von 250 bis 600 mOs/l liegt und die Viskosität nicht mehr als 9 Cp beträgt.

2. Präparat nach Anspruch 1, dadurch gekennzeichnet, daß der Immunglobulin-Gehalt 14 bis 16 % (G/V) beträgt.

3. Präparat nach Anspruch 1 oder 2, dadurch gekennzeichnet, daß die Viskosität 3 bis 8 cP beträgt.

4. Präparat nach einem der Ansprüche 1 bis 3, dadurch gekennzeichnet, daß es einen pH-Wert im Bereich von 4 bis 8 aufweist.

5. Präparat nach einem oder mehreren der Ansprüche 1 bis 4, dadurch gekennzeichnet, daß das Präparat zur Stabilisierung und/oder Einstellung seiner physikalischen Parameter Kohlenhydrate, insbesondere Saccharide, enthält.

6. Präparat nach Anspruch 5, dadurch gekennzeichnet, daß das Präparat als Saccharide Glucose oder Saccharose enthält.

7. Präparat nach einem oder mehreren der Ansprüche 1 bis 6, dadurch gekennzeichnet, daß es Immunglobuline in nativer Form enthält.

8. Präparat nach einem oder mehreren der Ansprüche 1 bis 7, dadurch gekennzeichnet, daß es zur weiteren Haltbarmachung in lyophilisierter Form vorliegt.

9. Präparat nach einem oder mehreren der Ansprüche 1 bis 7, dadurch gekennzeichnet, daß es als Flüssigpräparat oder in tiefgefrorener Form vorliegt.

10. Präparat nach einem oder mehreren der Ansprüche 1 bis 9, dadurch gekennzeichnet, daß es in einer durch eine Behandlung zur Virusinaktivierung erhältlichen, bezüglich von durch Blut übertragbarer infektioser Agentien sicheren Form vorliegt.

11. Verfahren zur Herstellung eines stabilen, hochkonzentrierten, intravenös verträglichen Immunglobulin-Präparates nach einem oder mehreren der Ansprüche 1 bis 9, dadurch gekennzeichnet, dass man eine Immunglobulin-Lösung mit einem Immunglobulin-Gehalt von 13,5 bis 17,5 % (G/V) herstellt, einen Kohlenhydrat-Gehalt sowie einen Gehalt an Sacchariden, der so zu wählen ist, dass die Osmolarität im Bereich von 250 bis 600 mOs/l liegt und eine Viskosität von nicht mehr als 9 Cp einstellt, und, wenn erwünscht, die erhaltene Formulierung anschließend in eine lyophilisierte oder tiefgefrorene Form überführt.

12. Verfahren nach Anspruch 11, dadurch gekennzeichnet, daß vor oder nach Einstellung des Immunglobulin-Präparates IgG-Aggregaten entfernt werden.

13. Verfahren nach Anspruch 11 oder 12 zur Herstellung eines Präparates nach Anspruch 10, dadurch gekennzeichnet, daß man zusätzlich eine Behandlung zur Virusinaktivierung durchführt.

## Claims

1. Stable, highly concentrated, intravenously acceptable immunoglobulin preparation, characterised in that the preparation has an immunoglobulin content of 13.5 to 17.5 % (W/V), the content of saccharides being selected so that the osmolarity lies in the range from 250 to 600 mOs/l and the viscosity is not more than 9 cP.

2. Preparation according to claim 1, characterised in that the immunoglobulin content is 14 to 16 % (W/V).

3. Preparation according to claim 1 or 2, characterised in that the viscosity is 3 to 8 cP.

4. Preparation according to one of claims 1 to 3, characterised in that it has a pH value in the range from 4 to 8.

5. Preparation according to one or more of claims 1 to 4, characterised in that the preparation contains carbohydrates, in particular saccharides, to stabilise and/or adjust its physical parameters.

6. Preparation according to claim 5, characterised in that the preparation contains glucose or saccharose as saccharides.

7. Preparation according to one or more of claims 1 to 6, characterised in that it contains immunoglobulins in native form.

8. Preparation according to one or more of claims 1 to 7, characterised in that it is present in lyophilised form for further preservation.

9. Preparation according to one or more of claims 1 to 7, characterised in that it is present as a liquid preparation or in deep-frozen form.

10. Preparation according to one or more of claims 1 to 9, characterised in that it is present in a form which is safe regarding infectious agents which can be transmitted by blood and which can be obtained by treatment for virus inactivation.

11. Process for producing a stable, highly concentrated, intravenously acceptable immunoglobulin preparation according to one or more of claims 1 to 9, characterised in that an immunoglobulin solution having an immunoglobulin content of 13.5 to 17.5 % (W/V) is produced, a carbohydrate content and a saccharides content being selected so that the osmolarity lies in the range from 250 to 600 mOs/l and a viscosity of not more than 9 cP is adjusted, and, if required, the formulation obtained is then converted to a lyophilised or deep-frozen form.

12. Process according to claim 11, characterised in that IgG aggregates are removed before or after adjustment of the immunoglobulin preparation.

13. Process according to claim 11 or 12 for producing a preparation according to claim 10, characterised in that a treatment for virusinactivation is additionally carried out.

## Revendications

1. Composition d'immunoglobuline tolérable par administration intraveineuse, fortement concentrée, stable, caractérisée en ce que la composition présente une teneur en immunoglobulines de 13,5 à 17,5 % (G/V, poids/volume), la teneur en saccharides étant choisie telle que l'osmolarité est située dans la plage de 250 à 600 mOs/l et la viscosité n'est pas supérieures à 9 cP.

2. Composition selon la revendication 1, caractérisée en ce que la teneur en immunoglobulines est de 14 à 16 % (G/V).

3. Composition selon la revendication 1 ou 2, caractérisée en ce que la viscosité est de 3 à 8 cP.

4. Composition selon l'une des revendications 1 à 3, caractérisée en ce qu'elle présente une valeur de pH comprise dans la plage de 4 à 8.

5. Composition selon une ou plusieurs des revendications 1 à 4, caractérisée en ce que la composition contient des hydrates de carbone, en particulier des saccharides, pour assurer la stabilisation et/ou le réglage de ses paramètres physiques.

6. Composition selon la revendication 5, caractérisée en ce que la composition contient, en tant que saccharides, du glucose ou du saccharose.

7. Composition selon une ou plusieurs des revendications 1 à 6, caractérisée en ce qu'elle contient des immunoglobulines sous forme native.

8. Composition selon une ou plusieurs des revendications 1 à 7, caractérisée en ce qu'elle se présente sous forme lyophilisée, dans le but d'augmenter sa durée de conservation.

9. Composition selon une ou plusieurs des revendications 1 à 7, caractérisée en ce qu'elle se présente sous forme de composition liquide, ou sous forme surgelée.

10. Composition selon une ou plusieurs des revendications 1 à 9, caractérisée en ce qu'elle se présente sous une forme sûre du point de vue des agents infectieux transmissibles par le sang, pouvant être obtenue par un traitement d'inactivation virale.

11. Procédé de préparation d'une composition d'immunoglobuline tolérable par administration intraveineuse, fortement concentrée, stable, selon une ou plusieurs des revendications 1 à 9, caractérisé en ce que l'on prépare une solution d'immunoglobuline, d'une teneur en immunoglobulines de 13,5 à 17,5 % (G/V), on règle une teneur en hydrate de carbone, ainsi qu'une teneur en saccharides, qui est choisie telle que l'osmolarité est située dans la plage de 250 à 600 mOs/l et une viscosité ne dépassant pas 9 cP et, si on le souhaite, la formulation obtenue est ensuite transférée sous une forme lyophilisée ou surgelée.

12. Procédé selon la revendication 11, caractérisé en ce que les groupes IgG sont éliminés, avant ou après réglage de la composition d'immunoglobuline.

13. Procédé selon la revendication 11 ou 12, par la préparation d'une composition selon la revendication 10, caractérisé en ce que, en plus, on effectue un traitement d'inactivation virale.
